# EUROPEAN PATENT SPECIFICATION

(11) **EP 3 124 016 B1**
(45) Date of publication and mention of the grant of the patent: **04.09.2019**
(21) Application number: 15460037.3
(22) Date of filing: 31.07.2015
(51) Int. Cl.: A61K 9/107, A61K 9/113, A61K 9/06, A61K 47/02, A61K 47/06, A61K 47/08, A61K 47/10, A61K 47/18, A61K 31/546, A61P 17/06

(54) **ANTIPSORIATIC EMULSION COMPOSITION COMPRISING CEFAZOLIN**
EMULSION MIT CEFAZOLIN GEGEN PSORIASIS
COMPOSITION D'ÉMULSION ANTI-PSORIASIQUE COMPRENANT DE LA CÉFAZOLINE

(43) Date of publication of application: 01.02.2017
(73) Proprietor: Warszawski Uniwersytet Medyczny, 02-091 Warszawa (PL)
(72) Inventor: Zegrocka-Stendel, Oliwia, 05-092 Tomianki (PL); Huszcza, Grzegorz, 03-988 Warszawa (PL); Pesta, Edyta, 02-495 Warszawa (PL); Smedra, Katarzyna, 03-193 Warszawa (PL); Koziak, Katarzyna, 02-708 Warszawa (PL)
(74) Representative: Lazewski, Marek

(56) References cited:
- EP-A1- 0 103 911
- EP-A1- 2 774 655
- WO-A1-91/08733
- WO-A1-98/26788
- WO-A1-2014/001353
- LASHMAR U T ET AL: "Correlation of rheological properties of an oil in water emulsion with manufacturing procedures and stability", INTERNATIONAL JOURNAL OF PHARMACEUTICS, ELSEVIER BV, NL, vol. 91, no. 1, 5 April 1993 (1993-04-05), pages 59-67, XP025557932, ISSN: 0378-5173, DOI: 10.1016/0378-5173(93)90421-B [retrieved on 1993-04-05]

## Description

### Field of the invention

The present invention relates to an antipsoriatic emulsion composition comprising cefazolin. The pharmaceutical composition is in the form of a cream for topical use on profuse exfoliating and cornifying skin sites, in particular on psoriatic lesions, eczema and atopic inflammatory changes of the skin.

### Background of the invention

Psoriasis is a chronic inflammatory disease of the skin of unknown etiology. Genetic as well as immunological factors play the essential role in the development of this illness. Psoriasis is characterized by the infiltration of neutrophils and lymphocytes into the dermis and impaired keratinocytosis (M.Abe, Journal of Dermatology 2006, 2: 108-111). Erythematous, scaly, sharply demarcated plaques of different size and shape, very often with concomitant itching, are the hallmarks of psoriasis.

Modern therapy of psoriasis includes topical and systemic treatment, phototherapy and miscellaneous methods. A routinely used topical treatment embraces exfoliating medications, such as ointments containing salicylic acid or urea-based ointments and creams, medicines reducing epidermal proliferation - mainly anthralins (Cignolin), wood tar as well as cytostatic drugs. The recent generation of anti-psoriatic topical medications comprises corticosteroids, retinoids and vitamin D₃ analogues. The systemic treatment embraces orally administered retinoids, immunomodulating drugs, such as cyclosporin A, or tacrolimus as well as cytostatic drugs, such as methotrexate and hydroxyurea. The current strategy for the psoriasis treatment and concomitant complications of the ailment is based on the application of biologics which exert immunosuppressive activity. This group of medications includes, for example, monoclonal antibodies - efalizumab and infliximab, alefacept and a recombinant receptor protein - etanercept indicated for the psoriatic arthritis management.

Some of these therapies proved to be inefficient or burdensome for patients. In general, the systemic treatment is associated with the risk of experiencing serious side effects. In addition, a biologic therapy is not always recommended due to the co-existing diseases, for instance. These disadvantages compel the use of the so called classical therapies of psoriasis as an alternate or intermittent treatment which may lead to frequent recurrences and significant decrease in the patient's quality of life (J.Szepietowski, Clinical Dermatology 2009, 11 (3)).

In the last decade, vitamin D₃ analogues have been used topically as the first-line treatment. The efficacy and safety of tacalcitol as well as the fact that in therapeutic doses it does not affect calcium homeostasis have been proved in clinical trials (P.C.M. van de Kerkhof, British Journal of Dermatology 2002: 146, 414-422).

Psoriasis is a chronic, relapsing inflammatory disease, therefore patients require long-term treatment of low cumulative toxicity potential, preferably. An optimal therapy is expected to provide a long-lasting efficacy, while being deprived of the long-term undesirable effects. It should also be well tolerated and accepted by the patients in order for them to follow their doctor's recommendations.

Therefore, the pursuit of a new, better anti-psoriatic therapy which will meet the aforementioned requirements is still ongoing.

The research findings of the screening trials disclosed in the PCT patent application WO 2015/044762, revealed a substance selectively binding and blocking the II-15-specific receptor sub-unit - IL-15Rα and simultaneously blocking the IL-2Rβ and IL-2Ry sub-units shared by IL-15 and IL-2, which results in the inhibition of the biological activity of these cytokines.

Interleukin 15 (IL-15) is a cytokine of pleiotropic activity affecting the cells of the immune system as well as other type cells. Due to its broad-spectrum activity, IL-15 is often placed at the top of the pro-inflammatory cytokine cascade. The impairment of natural mechanisms regulating the expression of IL-15, resulting in the overproduction of the cytokine, contributes directly to the development of inflammatory processes, autoimmune responses, infectious diseases and tumors. IL-15 is believed to be a key factor in psoriasis etiology (Villadsen L.S. et al., J. Clin. Invest. 112, 1571-80 (2003)).

A significant influence of IL-15 on the pathogenesis of psoriasis poses an opportunity for therapeutic intervention by blocking the biological activity of the cytokine. The validity of this approach has been proved experimentally. The inhibition of the IL-15 activity using a soluble IL-15Rα receptor (Liew F.Y., McInnes I.B., Ann. Rheum. Dis. 61 Supl. 2, ii100-2 (2002); Ruchatz H. et al., J Immunol. 160, 5664-60 (1998); Smith X.G. et al., J. Immunol. 165, 3444-50 (2000); Wei X et al., J. Immunol. 167, 277-82 (2001)), IL-2/IL-15Rβ receptor antibodies (Morris J.C., Proc. Natl. Acad. Sci. USA 103, 401-6 (2001); Tinubu S.A. et al., J. Immunol. 153, 4330-8 (1994)), anti-IL-15 antibodies (Villadsen L.S. et al. J. Clin. Invest. 112, 1571-80 (2003)) or a recombinant IL-15 molecule of competitive antagonist properties (Ferrari-Lacraz S. et al., J. Immunol. 173, 5818-26 (2004); Kim Y.S. et al., J. Immunol. 160, 5742-8 (1998)) resulted in the attenuation of IL-15-mediated disease symptoms. In experimental therapies using the mouse model psoriasis, alleviation of psoriatic signs has been observed (Villadsen L.S. et al. J. Clin. Invest. 112, 1571-80 (2003)). All therapeutic strategies based on the IL-15 inhibition seem equally effective, but vast majority of them has only been used under laboratory conditions so far.

As was proved in the *in vitro* study, the substance that apart from its broad spectrum of anti-bacterial activity effectively inhibits IL-15- and Il-2-induced biological responses is cefazolin ((6*R*,7*R*)-7-[[-2-amino-2-phenylacetyl]amino]-3-methyl-8-oxo-5-thia-1-azabicyclo[4.2.0]oct-2-ene-2-carboxylic acid) - first-generation cephalosporin antibiotic of proved medical efficacy.

Biological activity of cefazolin has been supported by *in vitro* studies, whose results have proved the efficacy of cefazolin in suppressing the proliferation of IL-15-stimulated cells and decreasing the Il-15-induced production of TNF-α and IL-17. The same results were obtained for Il-2-stimulated cells.

Because of that cefazolin may be effective in inhibiting IL-15 and IL-2-induced excessive cellular response by blocking the IL-15 specific IL-15Rα receptor as well as the IL-2Rβ and IL-2Ry receptor subunits shared by these two cytokines.

Despite the efficacy of action, the use of cefazolin in a wide-range treatment is limited due to some inconveniences resulting from its poor absorption from the digestive tract. This compound is well absorbed into the pleural cavity, peritoneal cavity, synovial fluid, bile, bones and urine, but does not penetrate into the cerebrospinal fluid. Currently available pharmaceutical cefazolin preparations are intended for parenteral, *i.m.* or *i.v*., administration.

As far as skin diseases such as psoriasis are concerned, topical application of pharmaceutical preparations is usually more effective than oral administration, injections and other systemic methods due to higher bioavailability and minor undesirable effects, such as the active substance penetration into the blood stream.

Pharmaceutical compositions intended for topical use comprise ointments, creams, lotions, transdermal patches, poultices, powders, aerosols etc. The most suitable dosage forms used in the treatment of skin diseases such as psoriasis are ointments and creams.

Accordingly, formulation studies have been carried out in order to develop a pharmaceutical composition containing cefazolin for topical use on skin lesions, characterized not only by high therapeutic activity, but also by the utility value.

Only a few patent applications exist that disclose pharmaceutical compositions containing cefazolin for topical use and in none of these publications data concerning stability of the product have been revealed. A limited number of such publications may well result from the difficulties in obtaining a pharmaceutical product of a long-term stability required for pharmacy medications. This effect is attributed to the nature of cefazolin as such, which is unstable in the media containing water, even at the temperature range 4-5°C.

The results of the studies of cefazolin stability in the aqueous solutions of eye drops have been disclosed *inter alia* in J. Clin. Pharm. and Therapy (1998), 23, 41-47 and Acta Pol. Pharm., vol. 69, 1, 95-105 (2012).

In J. Clin. Pharm. and Therapy the test results regarding the concentration of cefazolin sodium salt, pH and osmolarity of the eye drops composition containing 1% aqueous solution of the active substance in the presence of a preservative or preservative free, after the storage at 4°C and room temperature, have been disclosed. The eye drops stored at 4°C were stable for 42 days, while upon storage in room temperature the stability lasted only for a few days and the pH and osmolarity increase was observed. The authors of the Acta Pol. Pharm. paper examined the stability of 1% and 5% cefazolin aqueous solutions of eye drops at 4°C and 20°C, in different types of buffer solutions. The acetate buffer of pH 4.5 and 4.7, phosphate buffer of pH 7.5, the solutions of sodium chloride and glycerol were used in these tests. The researchers found that the stability of cefazolin in aqueous media is mainly influenced by pH and temperature and the highest stability of cefazolin was determined under acidic conditions.

Due to the low stability of cefazolin in aqueous solutions, the eye drops are not commercially available but might only be prepared directly before use in hospital eye units.

In the specification of CA 502542 A, the process for the preparation of an ointment containing a broad-spectrum activity antibiotic has been disclosed. The process embraces mixing of dispersing agents, lanolin, vaseline, wax, mineral oil and antibiotic, at such proportions that the ointments melting point ranges from 33 to 44°C. The composition of the ointment containing from 1 to 10% of the dispersing agents, glycerol monoester and long-chain fatty acid or cholesterol ester, from 5 to 20% of lanolin, from 10 to 30% of petrolatum, from 5 to 20% of wax selected among ceresin, paraffin and beeswax, from 30 to 60% of liquid paraffin and 0.1-10% of antibiotic, has been disclosed.

EP 0043738 B1 specification discloses a composition for topical use characterized by an enhanced transdermal absorbability, comprising a) from 0.01% to 35% by weight of the active substance selected from antibiotic agents, b) from 0 to 70% by weight of ethanol or isopropanol, c) from 0.01 to 99% by weight of the penetration-enhancing carrier consisting of an appropriate diol and the cell envelope-disordering compound selected from among the esters of myristic or lauric acids.

EP 0103911 A1 discloses an emollient-comprising composition for topical use. The claims relate to the emulsion composition comprising 9-89% of petrolatum, 0.25-10% of emulsifier (glyceryl monooleate, glyceryl monolinoleate or the mixture thereof), 10-90% of water and 0.01-25% of the active substance, for example antibiotic. It has been revealed in the example IV that cefazolin may be used as the ingredient of the composition.

In EP 0455396 B1, preparation of an osmotically balanced aqueous composition, liquid at room or lower temperature, becoming a gel at the temperature of a human body, intended for topical use comprising, among others, the polyoxyalkylene block copolymer, surfactant, polyether and the active substance, has been disclosed. In the description of the invention cefazolin has been revealed as the active ingredient.

In US 5409917 A, a composition of a cream for the treatment of acne containing an antibiotic selected from the group of cephalosporins, including cefazolin, etoxylated cetostearyl alcohol, alcohol and a fatty acid ester as well as the water phase comprising water-miscible alcohol, for example ethanol, isopropanol, propylene glycol, has been disclosed.

The specification of WO 01/01960 A1 reveals a composition in the form of a cream, for example, consisting of triglyceride, two surfactants including a hydrophilic one and the active substance dissolved in triglyceride, provided that the amounts of triglyceride and the surfactants are so adjusted as to form a clear dispersion when mixing with water.

WO 01/12155 A1 discloses a composition of enhanced hydrophilic absorption of the active substance, among others the cefazolin sodium salt, that also comprises two surfactants including a hydrophilic one.

WO 2007/103555 discloses a composition for topical use with pH in the range 3.0 to 7.4, consisting of two skin penetrating substances of synergistic activity selected among, for example, short-chain alkyl diols, C₁₀-C₂₀ fatty acids and esters thereof, substituted and unsubstituted C₄-C₂₀ alcohols and similar compounds as well as the active substance, for example, cefazolin.

US 2013184242 A1 relates to a pharmaceutical composition for, among others, topical use, showing rheological properties conferring the semi-solid form in steady-state and the liquid form when applied under the influence of shear forces. The composition consists of the vehicle containing from 75 to 99% of a hydrophobic solvent, for example olive oil, rapeseed oil, soybean oil, or similar as well as up to 25% of fatty alcohol as a solidifier and a biologically active ingredient, for example an antibiotic.

WO 2013/163734 A1 discloses a topical non-aqueous, non-exfoliating formulation including an effective tissue-penetrating organic solvent, for example C₁-C₁₀ alkyl sulfoxide, an organic co-solvent, for example glycol, poly-glycol, alcohol, a film-forming agent and the active substance, for example an antibiotic, including cefazolin.

For the preparation of the cream formulations for pharmaceutical use, water-in-oil (W/O) or oil-in-water (O/W) type emulsions are usually used consisting of a hydrocarbon or fatty ingredient, water and emulsifier in order to obtain an emulsion of required characteristics and high stability. Standard emulsion bases contain fatty components, such as white soft paraffin, liquid paraffin and/or liquid oils as triglycerides mixtures, emulgated in the water phase comprising water soluble buffers, chelating agents and preservatives.

Emulsions also contain moisturizing agents, skin penetration and absorption enhancers, softening agents, antioxidants as well as viscosity-regulating agents and emulsion stabilizers.

Topical formulations used in psoriasis treatment, apart from the proved pharmacological activity and safety when applied on skin lesions, for example on the patient's scalp, are also expected to display an appropriate stability upon storage and use. It is either important that the compositions, particularly those in the form of a liquid emulsion or a semi-solid cream, are characterized by an appropriate appearance and usefulness. It is essential that they are easily applicable, do not stick to hair and have an appropriate texture that prevents them from quickly running down.

Physicochemical stability is a significant problem when preparing these pharmaceutical formulations, due to the fact that the emulsions are susceptible to temperature changes and mechanical factors. Even minor modifications of the ingredients proportions or the addition of a non-compliant component may result in the decomposition of the emulsion's structure seen as phase separation or texture change during manufacturing or storage.

In their efforts to obtain a stable cefazolin emulsion, the Authors of the present invention faced the aforementioned problems resulting from the instability of the active ingredient in the water phase. Despite adding various buffer solutions disclosed in example 1 in a short period of time the water phase containing cefazolin became yellow, the color change being a visible sign of the active substance decomposition.

To overcome the problem of cefazolin's low stability, the Authors of the present invention modified the composition of the emulsion base by selecting appropriate oil ingredients, emulsifier and buffers. During the preformulation studies, the composition of the emulsions containing a nonionic emulsifier - polyoxyethylene ether of stearyl alcohol consisting of 21 ethylene oxide units (INCI Steareth-21) usually applied in the production of O/W type emulsions in the cosmetic preparations, was developed. Although the emulsions comprising the said emulsifier maintained their physical stability, they did not meet the essential requirement for an active pharmaceutical ingredient, i.e. long term stability in room temperature.

The fact that the Authors of the present invention have succeeded in developing a stable formulation consisting of an appropriate buffer solution and a simple oil phase comprising liquid paraffin or petrolatum, alkyl alcohol and nonionic emulsifier, may be viewed as serendipitous according to the state of the art.

The innovative approach adopted in the pharmaceutical technology for combining the standard ingredients provided a stable emulsion of an appropriate texture, usefulness and able to retain its stability at least at °C.

### Summary of the invention

The present invention relates to an antipsoriatic emulsion composition, comprising an effective therapeutic dose of cefazolin, the ingredients of (i) the oil phase and the ingredients of (ii) the water phase, characterized by that
(i) the ingredients of the oil phase include:
   - liquid paraffin or white petrolatum,
   - polyoxyethylene stearyl ether (21), and
   - cetostearyl alcohol;
(ii) the ingredients of the water phase include a buffer solution, consisting of trometamol (TRIS), boric acid and water, to maintain the pH value of the initial composition from 6 to 9,
(iii) comprising an additional amphiphilic nonionic emulsifier which is a condensation polymer of polyoxypropylene glycol and polyoxyethylene glycol, and other water soluble/miscible excipients, wherein the water content accounts for 50 to 80% by weight of the composition.

The other ingredients of the composition include as the water soluble/miscible excipients an organic solvent selected from a group of alcohols, diols and triols, preferably ethanol, isopropanol, propylene glycol or glycerin; phenoxyethanol and urea.

The emulsion is in the form of a cream containing preferably about 55 to 65% by weight of water.

Alternatively, the composition is in the form of a lotion, consisting of about 70 to 75% of water.

### Description of the invention

During preformulation works dealing with the development of the formulation for the emulsion containing cefazolin, it was observed that the composition of the emulsion base is not the only factor influencing oil phase dispersion in the water phase, or, in other words, the particle size of the oil phase. Additional factors are the pH value of the water phase and the method of emulsion preparation which substantially contribute to its physicochemical stability.

According to the present invention, an emulsion characterized by physical stability and appropriate texture can be obtained using liquid paraffin or white petrolatum, cetostearyl alcohol and polyoxyethylene stearyl ether (21) as nonionic emulsifiers in the oil phase.

Liquid paraffin as the mixture of liquid hydrocarbons is a common ingredient of emulsions used in pharmaceuticals and cosmetics due to its property to provide occlusive barrier to epidermal water loss and thus preventing the skin from over-drying. According to the invention, liquid paraffin or white petrolatum content accounts for 4.0 to 20.0% by weight of the total composition weight.

Stearyl alcohol etherified with 21 moles of ethylene oxide, obtained as the condensation product of the stearyl alcohol mixture and 20 moles of ethylene oxide (INCI: Steareth-21) is commercially available under the brand name Brij™ S721, for instance. It is a nonionic surfactant characterized by high equilibrium constant of the hydrophilic-lipophilic balance (HLB 16) which results from the combination of the lipophilic properties of stearyl alcohol and the unique arrangement of different length ethylene oxide units. It can be used in combination with various ingredients in a broad pH range as a substance decreasing the surface tension, a dispersing agent and an emulsifier.

According to the invention, the optimal amount of the nonionic surfactant in the composition accounts for 3 to 10% by weight, preferably 7 to 9%, of the total composition weight.

Cetostearyl alcohol is the mixture of solid aliphatic alcohols consisting mainly of stearyl alcohol (C₁₈H₃₈O) and cetyl alcohol (C₁₆H₃₄O), possibly with some addition of other alcohols, for example myristyl alcohol which exhibits the properties of an emollient, emulsifier and viscosity increasing agent. According to the invention, its content accounts for 2.0 to 10.0% of the total composition weight.

The experiments proved that the choice of a suitable buffer solution is the most essential factor in the preparation of stable emulsions containing cefazolin. It has been discovered that best stability of the compositions according to the invention can be achieved by using the TRIS (trometamol)/boric acid buffer (pH about 8.7).

The adjustment of the amounts of other ingredients of the composition is up to those skilled in the art. The appropriate excipients are disclosed, e.g., in the Handbook of Pharmaceutical Excipients, R.C.Rowe, P.J.Sheskey, S.C.Owen, ed. 5th, 2006, American Pharmaceutical Association Washington and The Pharmaceutical Press London.

Apart from the buffer solutions, the water phase may also comprise other water soluble excipients, such as phenoxyethanol and urea which are used in pharmaceutical practice on a regular basis.

Phenoxyethanol, used as a preservative, provides an effective microbiological protection of the emulsion according to the invention. Due to the substantial water content it can be used in the amount of at least 0.75%, preferably 1.0% of the total composition weight. This amount is in line with the recommendations and remains within the acceptable range disclosed in 5.1.3 Ph Eur. The efficacy of such an amount of the preservative was proved by suitable tests performed in compliance with the European Pharmacopoeia (Ph Eur.). The chosen amount of the preservative does not affect the stability of the emulsion.

Urea, a diamide of the carbonic acid, the moisturizing compound naturally occurring in the skin, is also known as an anti-microbial and anti-pruritus agent. Its proven anti-inflammatory properties result from its anti-proliferation and anti-granulation activity. Urea is used as a standard component in cosmetic products as a water-binding substance (humectant), anti-microbial and exfoliating agent. At higher concentrations, urea contributes to skin softening and enhanced transdermal permeability which enable deeper dermal penetration of other ingredients. It is also applied as an exfoliating agent. Its water-binding ability reduces the bacterial growth rates; consequently, urea-containing cosmetic products may comprise other preservatives at lower concentrations. Usually, the amount of urea in emulsions accounts for 3.0 to 7.0% by weight, preferably 5% by weight of the total composition weight.

Preferably, the emulsion composition according to the invention comprises also a water miscible organic solvent selected from a group including alcohols, diols and triols, preferably ethanol, isopropanol, propylene glycol or glycerol. Most preferably, the solvent of choice is propylene glycol due to its water-binding and softening properties. It also facilitates the absorption of the active ingredient. Its content in the composition may be in the range from 2.0 to 10.0%.

An anti-psoriatic emulsion containing 2 g or 5 g of cefazolin sodium salt and the following excipients expressed in weight percentage concentration, calculated per 100 g of the composition, is disclosed for reference purposes:

| **Ingredient** | **Content (% by weight)** |
|---|---|
| Liquid paraffin | 4.0-20.0 |
| Polyoxyethylene stearyl ether (21) | 3.0-10.0 |
| Cetostearyl alcohol | 2.0-10.0 |
| Organic solvent | 2.0-10.0 |
| Phenoxyethanol | 0.0-1.0 |
| Urea | 3.0-7.0 |
| Buffer solution | up to pH 6 - 9 |
| Water qs to | 100 % |

This emulsion composition, which is not according to the invention as claimed, is prepared following standard methods known in the pharmaceutical practice, by obtaining the water phase containing water soluble excipients (a preservative, a buffer solution) and heating it to 70-80°C. After careful stirring and heating to 65-75°C, the previously prepared oil phase is added to the water phase. The obtained mixture is stirred, cooled down, and then subjected to homogenization to form an appropriate structure of the emulsion, upon which the cooling is continued and the solvent selected from a group of alcohols, diols and triols is added, followed in the end by the active substance - cefazolin sodium salt dissolved in the second part of water. The mixture is homogenized in order to obtain the active substance dispersed equally throughout. After the emulsion composition cools down to room temperature, the product is dosed and packed into unit packages.

Unexpectedly, it was discovered that the introduction of an additional nonionic emulsifier of an amphiphilic structure into the emulsion composition together with the change of the emulsion production technology not only provide physically stable emulsions but also help prevent the decomposition of naturally unstable cefazolin substance during storage.

This additional emulsifier in the composition according to the invention is a condensation polymer of polyoxypropylene glycol and polyoxyethylene glycol, preferably the one commercially available under the brand name Poloxamer 188. Due to its amphiphilic structure, the polymer is well soluble in water, increases mutual miscibility of the substances of different hydrophobicity and shows suitable surface-active properties.

In this particular embodiment of the invention the antipsoriatic emulsion composition contains 2 g or 5 g of cefazolin sodium salt and the following excipients expressed in weight percentage concentration, calculated per 100 g of the composition:

| **Ingredient** | **Phase** | **Content (% by weight)** |
|---|---|---|
| Liquid paraffin | | 10.0-20.0 |
| Polyoxyethylene stearyl ether (21) | (1) | 5.0-10.0 |
| Cetostearyl alcohol | | 2.0-6.0 |
| Cefazolin | | 2.0-5.0 |
| Organic solvent | (2) | 4.0-7.0 |
| Buffer solution | | up to pH 6 - 9 |
| Polymer of polyoxypropylene glycol and polyoxyethylene glycol | | 1.0-2.0 |
| Urea | (3) | 3.0-7.0 |
| Phenoxyethanol | | 0.0-1.0 |
| Water, qs to | | 100 % |

wherein the water content accounts for 50 to 80% by weight of the composition, and the buffer solution consists of TRIS, boric acid and water. Introduction of an additional emulsifier allows to employ a different method of the emulsion production, upon which the active substance is added into the composition not at the end of the process of the emulsion formation, but by emulsifying the first buffered water phase (2) containing the active ingredient and the oil phase (1), then by emulsifying the obtained system with the second water phase comprising an additional emulsifier (3) and adding the remaining water soluble/miscible excipients.

In this particular embodiment of the invention, the emulsion composition is prepared as follows:
the first water phase (2) containing water soluble excipients, which are an organic solvent selected from a group of alcohols, diols and triols, and the ingredients of the buffer solution, is heated up to 70-80°C while stirring,
the previously prepared oil phase (1) is added at 70°C, the resulting mixture is stirred and cooled down to 50-60°C,
the second water phase (3) containing an additional amphiphilic nonionic emulsifier, which is a condensation polymer of polyoxypropylene glycol and polyoxyethylene glycol, and the remaining water soluble/water miscible excipients is introduced upon stirring,
the obtained composition is homogenized while maintaining the temperature at 50°C, until the active substance has dispersed uniformly,
the product is cooled down to room temperature, dosed and packed into unit packages.

The emulsion preparation method in this particular embodiment of the invention results in the formation of a "double" emulsion. The use of a suitable buffer solution and the modification of the emulsion preparation technology provides an appropriately buffered aqueous medium and oil phase surrounding the pH sensitive active substance which protect it from decomposing in the presence of water.

The cefazolin emulsion composition according to the present invention may be produced in the form of a lotion or a cream. The developed composition of the emulsion allows to obtain a product in a desired pharmaceutical form, from liquid to semi-solid, without significant changes of its content and proportions of particular ingredients. It relies on the use of different quantities of water, the latter being compensated by the presence of the oil phase components with no adverse effects on the stability and physicochemical parameters of the emulsion.

The emulsion composition in the form of a lotion contains about 70 to 75% by weight of water.

The emulsion composition in the form of a cream contains about 55 to 65% by weight of water.

The stability of the emulsion composition according to the present invention was tested at 5°C and 25°C/60% RH. The emulsion was characterized by good stability when stored at 5°C, but at higher temperatures the decomposition of the active substance of up to 50%, was observed after one month of storage.

Combining the therapeutic effects of the active substance - cefazolin, with the softening, moisturizing and exfoliating properties provided by the components of the cream or lotion base when applied on skin lesions results in beneficial antipsoriatic effects of the pharmaceutical composition according to the invention.

The emulsion composition according to the invention may be effectively used in the treatment of cell overproliferation-based skin diseases, such as psoriasis vulgaris, guttate psoriasis, erythrodermic psoriasis, psoriasis arthropatica, psoriasis inveterata and other diseases.

The present invention is illustrated by the following examples.

Comparative examples are provided only for better understanding of the invention.

### Examples

### Example 1. Selection of the buffer solution

In order to select the most suitable pH value of the emulsion formulation, standard aqueous solutions of cefazolin sodium salts (2% by weight) in the following buffer solutions were prepared:

**1. TRIS/boric acid buffer**

| Composition | mg/ml | pH |
|---|---|---|
| Trometamol | 0.42 | 8.7 |
| Boric acid | 0.2 | |

**2. Phosphate/citrate buffer (comparative example)**

| Composition | mg/ml | pH |
|---|---|---|
| Na₂HPO₄ x 7 H₂O | 22.08 | 7.19 |
| Citric acid | 1.855 | |

**3. Phosphate buffer (comparative example)**

| Composition | mg/ml | pH |
|---|---|---|
| NaH₂PO₄H₂O | 1.6 | 6.15 |
| Na₂HPO₄ | 0.25 | |

**4. HEPES (comparative example)**

| Composition | mg/ml | pH |
|---|---|---|
| HEPES | 238 | 7.43 |
| NaOH | up to pH ca. 7.4 | |

On account of the fast decomposition of the active substance in the phosphate-citrate and HEPES buffers, visibly detected by the mixture color change from white to yellow, TRIS/boric acid and phosphate buffers were used in further preformulation studies.

### Comparative example 2. Emulsion with the phosphate buffer

The emulsions containing 2% (CF 2%) or 5% (CF 5%) of cefazolin sodium salt and the phosphate buffer were prepared for the stability studies, their composition was as follows:

**CF 2%**

| **No.** | **Ingredient** | **Amount per 100 g [g]** |
|---|---|---|
| 1 | White petrolatum | 4 |
| 2 | Cetostearyl alcohol | 3 |
| 3 | BRIJ S721 | 3 |
| 4 | Urea | 5 |
| 5 | NaH₂PO₄ x H₂O | 0.16 |
| 6 | Na₂HPO₄ | 0.025 |
| 7 | Phenoxyethanol | 1 |
| 8 | Water I + II | 71.815 (56.815+15) |
| 9 | Propylene glycol | 10 |
| 10 | Cefazolin | 2 |
| | Total amount | 100 |

**CF 5%**

| **No.** | **Ingredient** | **Amount per 100 g [g]** |
|---|---|---|
| 1 | White petrolatum | 4 |
| 2 | Cetostearyl alcohol | 3 |
| 3 | BRIJ S721 | 3 |
| 4 | Urea | 5 |
| 5 | NaH₂PO₄ x H₂O | 0.16 |
| 6 | Na₂HPO₄ | 0.025 |
| 7 | Phenoxyethanol | 1 |
| 8 | Water I + II | 68.815 (53.815+15) |
| 9 | Propylene glycol | 10 |
| 10 | Cefazolin | 5 |
| | Total amount | 100 |

### Preparation of the water phase

The ingredients 4, 5, 6, 7, 9 and water 8 (portion I) were placed in a vessel, the mixture was stirred while being simultaneously heated until all components have dissolved. The solution was stirred using a mechanical anchor stirrer at 40 rpm, to reach 70°C temperature.

### Preparation of the oil phase

The ingredients 1, 2, 3 were placed in a glass beaker, the mixture was stirred while being simultaneously heated until all components have dissolved. The solution was stirred occasionally to reach 70°C temperature.

### Preparation of the cefazolin solution

To a glass beaker placed on a magnetic stirrer, water 8 (portion II) and cefazolin sodium salt (10) were added in the said order, while stirring at the rate of 500±100 rpm, until the entire substance has dissolved.

### Combining of the phases

The oil phase was stirred into a vessel containing the water phase. The resulting mixture was stirred at the rate of 40 rpm at 70°C for 20 min. It was cooled down upon stirring at a lower rate. When temperature reached about 60°C, stirring was reduced to 30 rpm and the mixture was subjected to homogenization at the rate of 13500 rpm for 15 min. After cooling down to 40°C, propylene glycol (9) was added to the obtained base solution and the resulting mixture was stirred at the rate of 40 rpm for 5 min.

### Addition of the solution containing the active substance

Cefazolin sodium salt solution (10) was added to the base solution. The obtained mixture was homogenized at 13500 rpm and stirred with a mechanical anchor stirrer at the rate of 30 rpm for 10 min at 40°C.

### Dosing

When homogenization was complete, the emulsion was cooled down to about 30°C. The cream was dosed into aluminum tubes.

### Emulsion stability studies

The emulsion compositions prepared according to comparative example 2 were chosen for the stability studies.

### Method of the stability studies

To assess the emulsion stability, the amount of the active substance - cefazolin was determined in the cream after storage for one month at two different temperatures, 5°C and 25°C/60% RH, as was the pH of the emulsion after storage at the same temperatures.

### Determination of the amount of the active substance:

The amount of the active substance was determined by high-performance liquid chromatography (HPLC), using a mobile phase: acetonitrile/water 0.1% formic acid (45:55 v/v), on a chromatography column (250/4.6 mm, 5µm) from Waters. In order to perform the HPLC analysis, the active substance was extracted from the emulsion using the mixture of acetonitrile and water 3:2 (v/v) during 30 min (sonication).

Regardless of the reproducibility of the isolation method, the cefazolin amount in the emulsion did not reach the initial values, 20 mg/g and 50 mg/g. On the chromatograms of the analyzed samples an increase in the additional peak (impurity) was observed in comparison to the additional peak on the cefazolin standard solution chromatogram. A 30% decrease in the surface area of the cefazolin main peak was noted when the standard solution was refrigerated (+5°C) for 48 h and a 60% decrease when it was kept in an autosampler at 25°C for 48 h.

The results of two batches of the emulsions (2% and 5%) tested immediately after preparation and when stored for 1, 3 and 6 (7) months at 5°C/60% RH and for 1 month at 25°C/60% RH, are collected in the tables below.

**Temp. 5°C**

| | **Cefazolin content [mg/g]** | | | | |
|---|---|---|---|---|---|
| | **1** | **2** | **3** | **4** | **5** |
| **BATCH** | **Theoretical amount** | **Time "0"** | **After 1 month** | **After 3 months** | **After 7 months** |
| CF-2 | 20 | 15.88 | --- | 15.83 | 13.13 |
| CF-5 | 50 | 39.29 | 43.6 | 38.76 | 27.95 |

**Temp. 25°C**

| | **Cefazolin content [mg/g]** | | |
|---|---|---|---|
| | **1** | **2** | **3** |
| **BATCH** | **Theoretical amount** | **Time "0"** | **After 1 month** |
| CF-2 | 20 | 15.88 | 7.15 |
| CF-5 | 50 | 39.29 | 17.18 |

The cefazolin concentration in the emulsion analyzed by HPLC immediately after the composition preparation remained in the range of 75-80% of the initial value. After the emulsion was stored for one month at 25°C, a significant decrease in the amount of the active substance (below 50% of its initial value) was observed. This drop in the active substance concentration was accompanied by the color change of the cream which became yellow.

The samples stored at 5°C were stable for 3-6 months.

### Example 3

### The emulsion with the TRIS/boric acid buffer

Five test creams A - E of different compositions, prepared under diverse technological conditions, were examined in order to assess the usefulness of the TRIS/boric acid buffer to maintain the stability of the emulsion. The buffer solution was prepared separately and a required amount was introduced into the composition:

| **BUFFER** | **mg/ml** |
|---|---|
| Trometamol (TRIS) | 0.42 |
| Boric acid | 0.2 |

**CREAM A (comparative example)**

| **Ingredient** | **Amount per 100 g [g]** | **Phase** |
|---|---|---|
| Liquid paraffin | 14 | (1) |
| Cetostearyl alcohol | 8 | |
| Brij S721 | 8 | |
| Propylene glycol | 2.4 | (2) |
| TRIS/boric acid buffer | 5.6 | |
| Cefazolin | 2 | |
| Water | 55 | (3) |
| Urea | 5 | |

**CREAM B (comparative example)**

| **Ingredient** | **Amount per 100 g [g]** | **Phase** |
|---|---|---|
| Liquid paraffin | 14 | (1) |
| Cetostearyl alcohol | 8 | |
| Brij S721 | 8 | |
| Propylene glycol | 8 | (2) |
| TRIS/boric acid buffer | 2 | |
| Cefazolin | 5 | (3) |
| Water | 55 | |

**CREAM C (comparative example)**

| **Ingredient** | **Amount per 100 g [g]** | **Phase** |
|---|---|---|
| Liquid paraffin | 14 | (1) |
| Cetostearyl alcohol | 8 | |
| Brij S721 | 8 | |
| Propylene glycol | 8 | (2) |
| TRIS/boric acid buffer | 2 | |
| Cefazolin | 5 | (3) |
| Water | 55 | |

**CREAM D**

| **Ingredient** | **Amount per 100 g [g]** | **Phase** |
|---|---|---|
| Liquid paraffin | 14 | (1) |
| Cetostearyl alcohol | 8 | |
| Brij S721 | 8 | |
| Propylene glycol | 2.4 | (2) |
| TRIS/boric acid buffer | 5.6 | |
| Cefazolin | 2 | |
| Water | 53.5 | (3) |
| Poloxamer 188 | 1.5 | |
| Urea | 5 | |

**CREAM E (comparative example)**

| **Ingredient** | **Amount per 100 g [g]** | **Phase** |
|---|---|---|
| Liquid paraffin | 14 | (1) |
| Cetostearyl alcohol | 8 | |
| Brij S721 | 8 | |
| Propylene glycol | 8 | (2) |
| Cefazolin | 2 | |
| Water | 53.5 | (3) |
| Poloxamer 188 | 1.5 | |
| Urea | 5 | |

In the cream batches A, B and C the influence of the buffer, glycol, as well as the mixture of the buffer and propylene glycol on cefazolin's stability were tested. In the cream batches D and E an additional emulsifier - Poloxamer 188 was introduced into the water phase (3). The compositions of the tested emulsions are presented collectively in the table below.

| **Ingredient** | **A** | **B** | **C** | **D** | **E** |
|---|---|---|---|---|---|
| Liquid paraffin | X | X | X | X | X |
| Cetostearyl alcohol | X | X | X | X | X |
| Brij S721 | X | X | X | X | X |
| Propylene glycol | X | X | X | X | X |
| Cefazolin | X | X | X | X | X |
| TRIS/boric acid buffer | X | X | X | X | --- |
| Water | X | X | X | X | X |
| Poloxamer 188 | --- | --- | --- | X | X |
| Urea | X | X | X | X | X |

### Preparation of the oil phase (1)

The ingredients of the phase (1): liquid paraffin, Brij S721 and cetylostearyl alcohol were gradually melted at 70-75°C, while being stirred with an anchor stirrer.

### Preparation of the water phase (2)

The ingredients of the phase (2): cefazolin and propylene glycol were placed in a beaker and dissolved in the water buffer, then heated up to 70°C and stirred on a magnetic stirrer. In the preparation E the buffer solution was substituted with propylene glycol.

### Preparation of the water phase (3)

The ingredients of the phase (3): urea and Poloxamer 188 (if present in the composition) were placed in a beaker and dissolved in water, heated up to 50°C and stirred on a magnetic stirrer.

### Combining of the phases

The phases (1) and (2) were mixed and stirred with a mechanical anchor stirrer, the resulting emulsion was homogenized for 10 min at the rate of 13500 rpm, while maintaining the temperature at 70°C. After cooling the emulsion down to 50-60°C, the phase (3) was introduced by stirring with a mechanical anchor stirrer, then it was homogenized for 10 min at the rate of 9500 rpm, at about 50 °C. The emulsion was cooled down to 30-40°C and dosed into PE unit containers.

The cefazolin amount and the pH value of the emulsion were determined after storage for 1 month in different temperatures. The results are presented in the table below.

| | **Cefazolin amount [mg/g]** | |
|---|---|---|
| | **1** | **2** |
| **Composition** | **1 month at 5°C** | **1 month at 25°C** |
| A | 14.35 | 5.43 |
| B | 11.72 | 5.08 |
| C | 15.44 | 5.15 |
| D | 19.56 | 5.63 |
| E | 13.68 | 4.82 |

## Claims

1. An antipsoriatic emulsion composition comprising an effective therapeutic dose of cefazolin, the ingredients of (i) the oil phase and the ingredients of (ii) the water phase, **characterized by** that
(i) the ingredients of the oil phase include:
- liquid paraffin or white petrolatum,
- polyoxyethylene stearyl ether (21), and
- cetostearyl alcohol;
(ii) the ingredients of the water phase include a buffer solution, consisting of trometamol (TRIS), boric acid and water, to maintain the pH value of the initial composition from 6 to 9,
(iii) comprising an additional amphiphilic nonionic emulsifier, which is a condensation polymer of polyoxypropylene glycol and polyoxyethylene glycol, and other water soluble/ miscible excipients, wherein the water content accounts for 50 to 80% by weight of the composition.

2. The emulsion composition according to claim 1, **characterized by** that the water soluble/miscible excipients comprise an organic solvent selected from a group of alcohols, diols and triols and/or phenoxyethanol and/or urea.

3. The emulsion composition according to claim 2, **characterized by** that the organic solvent is ethanol, isopropanol, propylene glycol or glycerin.

4. The emulsion composition according to claim 3, **characterized by** that the organic solvent is propylene glycol.

5. The emulsion composition according to any claim 1 - 4, **characterized by** that it is in the form of a cream containing from 55 to 65% by weight of water.

6. The emulsion composition according to claim 5, **characterized by** that it is in the form of a cream containing the following ingredients expressed in % by weight of the total composition weight:
| Ingredient | Phase | Content (% by weight) |
|---|---|---|
| Liquid paraffin | (1) | 10.0-20.0 |
| Polyoxyethylene stearyl ether (21) | | 5.0-10.0 |
| Cetostearyl alcohol | | 2.0-6.0 |
| Cefazolin | (2) | 2.0-5.0 |
| Organic solvent | | 4.0-7.0 |
| Buffer solution | | to pH 6 - 9 |
| Polymer of polyoxypropylene glycol with polyoxyethylene glycol | (3) | 1.0-2.0 |
| Urea | | 3.0-7.0 |
| Phenoxyethanol | | 0.0-1.0 |
| Water qs to | | 100 |

7. The process for the preparation of the emulsion composition according to claim 6, **characterized by** that
the first water phase (2) containing water soluble excipients, which are an organic solvent selected from a group of alcohols, diols and triols, and the ingredients of the buffer solution, consisting of trometamol (TRIS), boric acid and water, is heated up to 70-80°C while stirring, the previously prepared oil phase (1) is added at 70°C, the resulting mixture is stirred and cooled down to 50-60°C,
the second water phase (3) containing an additional amphiphilic nonionic emulsifier, which is a condensation polymer of polyoxypropylene glycol and polyoxyethylene glycol, and the remaining water soluble/water miscible excipients is introduced upon stirring, the obtained composition is homogenized while maintaining the temperature at 50°C, until the active substance has dispersed uniformly,
the product is cooled down to room temperature, dosed and packed into unit packages.

## Patentansprüche

1. Antipsoriatische Emulsionszusammensetzung, enthaltend eine wirksame therapeutische Dosis von Cefazolin, Ölphasenbestandtaile (i) und Wasserphasenbestantaile (ii), **dadurch gekennzeichnet, dass**:
(i) die Ölphasenbestandtaile:
- flüssiges Paraffin oder weißes Petrolatum,
- Polyoxyethylenstearylether (21) und
- Cetostearylalkohol
umfassen;
(ii) die Wasserphasenbestantaile eine Pufferlösung, bestehend aus Trometamol (TRIS), Borsäure und Wasser, umfassen, um den pH-Wert der anfänglichen Zusammensetzung von 6 bis 9 aufrechthalten,
(iii) umfassend einen zusätzlichen amphiphilen nichtionischen Emulgator, der ein Kondensationspolymer aus Polyoxypropylenglykol und Polyoxyethylenglykol und anderen löslichen/mit Wasser mischbaren Exzipientien ist, wobei der Wassergehalt 50 bis 80 Gew.-% der Zusammensetzung ausmacht.

2. Emulsionszusammensetzung gemäß Anspruch 1, **dadurch gekennzeichnet, dass** die wasserlöslichen/mischbaren Exzipientien ein organisches Lösungsmittel enthalten, das aus einer Gruppe von Alkoholen, Diolen und Triolen und/oder Phenoxyethanol und/oder Harnstoff ausgewählt wurde.

3. Emulsionszusammensetzung gemäß Anspruch 2, **dadurch gekennzeichnet, dass** das organische Lösungsmittel Ethanol, Isopropanol, Propylenglykol oder Glycerin ist.

4. Emulsionszusammensetzung gemäß Anspruch 3, **dadurch gekennzeichnet, dass** das organische Lösungsmittel Propylenglykol ist.

5. Emulsionszusammensetzung gemäß irgendeinem der Ansprüche 1 bis 4, **dadurch gekennzeichnet, dass** sie in Form einer Creme vorliegt, die 55 bis 65 Gew.-% Wasser enthält.

6. Emulsionszusammensetzung gemäß Anspruch 5, **dadurch gekennzeichnet, dass** sie in Form einer Creme vorliegt, enthaltend die folgenden Bestandteile ausgedrückt in Gew.-% des gesamten Zusammensetzungsgewichts:
| Bestandteil | Phase | Gehalt (in Gew.-%) |
|---|---|---|
| Flüssiges Paraffin | (1) | 10,0-20,0 |
| Polyoxyethylenstearylether (21) | | 5,0-10,0 |
| Cetostearylalkohol | | 2,0-6,0 |
| Cefazolin | (2) | 2,0-5,0 |
| Organische Lösung | | 4,0-7,0 |
| Pufferlösung | | auf pH 6 - 9 |
| Polymer aus Polyoxypropylenglykol und | (3) | 1,0-2,0 |
| Polyoxyethyleng lykol | | |
| Harnstoff | | 3,0-7,0 |
| Phenoxyethanol | | 0,0-1,0 |
| Wasser qs bis | | 100 |

7. Verfahren zur Herstellung der Emulsionszusammensetzung gemäß Anspruch 6, dadurch geknzeichnet, dass:
die erste Wasserphase (2), enthaltend wasserlösliche Exzipientien, die organische Lösungsmittel sind, ausgewählt aus einer Gruppe von Alkoholen, Diolen und Triolen, sowie die Bestandteile der Pufferlösung, bestehend aus Trometamol (TRIS), Borsäure und Wasser, unter Rühren auf 70-80°C erhitzt wurden,
die zuvor hergestellte Ölphase (1) bei 70°C zugegeben, die resultierende Mischung wurde gerührt und auf 50-60°C abgekühlt.
die zweite Wasserphase (3), die einen zusätzlichen amphiphilen nichtionischen Emulgator enthält, welcher ein Kondensationspolymer aus Polyoxypropylenglykol und Polyoxyethylenglykol sowie anderen wasserlöslichen/mit Wasser mischbaren Exzipientien ist, wurde beim Rühren eingeführt,
die so erhaltene Zusammensetzung wurde homogenisiert, wobei die Temperatur bei 50°C gehalten wurde, bis der Wirkstoff gleichmäßig dispergiert,
das Produkt wurde auf Raumtemperatur abgekühlt, dosiert und verpackt.

## Revendications

1. Composition d'émulsion antipsoriasique comprenant une dose thérapeutique efficace de céfazoline, les ingrédients de (1) la phase huileuse et les ingrédients de (2) la phase aqueuse, **caractérisée par le fait que**
(1) les ingrédients de la phase huileuse comprennent:
- paraffine liquide ou vaseline blanche,
- polyoxyéthylène stéaryl éther (21), et
- alcool cétostéarylique;
(2) les composants de la phase aqueuse comprennent une solution tampon composée de trométamol (TRIS), d'acide borique et d'eau, afin de maintenir le pH de la composition initiale de 6 à 9,
(3) comprenant un émulsifiant non ionique amphiphile supplémentaire, qui est un polymère de condensation de polyoxypropylène glycol et de polyoxyéthylène glycol et d'autres excipients solubles / miscibles à l'eau, dans lesquels la teneur en eau représente 50 à 80% en poids de la composition.

2. Composition d'émulsion selon la revendication 1, **caractérisée en ce que** les excipients solubles / miscibles dans l'eau comprennent un solvant organique choisi dans le groupe des alcools, des diols et des triols et / ou du phénoxyéthanol et / ou de l'urée.

3. Composition d'émulsion selon la revendication 2, **caractérisée en ce que** le solvant organique est l'éthanol, l'isopropanol, le propylène glycol ou la glycérine.

4. Composition d'émulsion selon la revendication 3, **caractérisée en ce que** le solvant organique est le propylène glycol.

5. Composition d'émulsion selon l'une quelconque des revendications 1 à 4, **caractérisée en ce qu'**elle se présente sous forme de crème contenant de 55 à 65% en poids d'eau.

6. Composition d'émulsion selon la revendication 5, **caractérisée en ce qu'**elle se présente sous la forme d'une crème contenant les ingrédients suivants, exprimée en % en poids du poids total de la composition:
| ingrédients | phase | contenu (% en poids) |
|---|---|---|
| Paraffine liquide | | 10,0-20,0 |
| Polyoxyéthylène stéaryl éther | (1) | 5,0-10,0 |
| Alcool cétostéarylique | | 2,0 à 6,0 |
| Céfazoline | | 2,0-5,0 |
| Solvant organique | (2) | 4,0-7,0 |
| Solution tampon | | jusqu'à pH 6 - 9 |
| Polymère de polyoxypropylène glycol avec polyoxyéthylène glycol | (3) | 1,0-2,0 |
| Urée | | 3,0-7,0 |
| Phenoxyethanol | | 0,0-1,0 |
| Eau | | qsp à 100 |

7. Procédé de préparation de la composition d'émulsion selon la revendication 6, **caractérisé en ce que**
la première phase aqueuse (2) contenant des excipients solubles dans l'eau, qui sont un solvant organique choisi dans un groupe d'alcools, de diols et de triols, et les ingrédients de la solution tampon, consistant en trométamol (TRIS), acide borique et eau, sont chauffés à 70-80°C sous agitation, la phase huileuse (1) préalablement préparée est ajoutée à 70°C, le mélange résultant est agité et refroidi à 50-60°C,
la deuxième phase aqueuse (3) contenant un émulsifiant non ionique amphiphile supplémentaire qui est un polymère de condensation de polyoxypropylène glycol et de polyoxyéthylène glycol et les excipients restants hydrosolubles / miscibles à l'eau est introduite sous agitation, la composition obtenue est homogénéisée en maintenant la température à 50°C, jusqu'à ce que la substance active se soit uniformément dispersée, le produit est refroidi à la température ambiante, dosé et emballé dans des emballages unitaires.
